# EUROPÄISCHE PATENTSCHRIFT

(11) **EP 0 911 332 B1**
(45) Veröffentlichungstag und Bekanntmachung des Hinweises auf die Patenterteilung: **14.04.2004**
(21) Anmeldenummer: 98811071.4
(22) Anmeldetag: 26.10.1998
(51) Int. Cl.: C07D 471/16, C07D 495/22, C07D 491/22, C07D 471/22, C09B 57/08, C09B 57/12, C08K 5/3447

(54) **Heterocyclische Verbindungen**
Heterocyclic compounds
Composés hétérocycliques

(30) Priorität: 27.10.1997 DE 19747395
(43) Veröffentlichungstag der Anmeldung: 28.04.1999
(73) Patentinhaber: Clariant Finance (BVI) Limited, Road Town, Tortola (VG)
(72) Erfinder: Boeglin, Patrick, 68170 Rixheim (FR); Kaul, Bansi Lal, 4105 Biel-Benken (CH); Piastra, Bruno, 68330 Huningue (FR)
(74) Vertreter: D'haemer, Jan Constant

(56) Entgegenhaltungen:
- CH-A- 561 763
- DD-A- 211 456
- DE-A- 2 451 049
- FR-A- 2 620 713

## Beschreibung

Die vorliegende Erfindung betrifft neue Farbmittel zur Kunststoffmassefärbung. Das Massefärben insbesondere von polaren Kunststoffen, wie z.B. Polyamiden, Polyestern, Polycarbonaten und ABS, stellt an die verwendeten Farbstoffe hohe Anforderungen hinsichtlich ihrer Hitzestabilität und Lichtechtheit.

In FR-A-2620713 werden heterocyclische Verbindungen zum Einfärben von Polyamiden offenbart, die in CH-A-561763 bereits als Pigmente beschrieben wurden. Die dort beschriebenen Farbmittel besitzen jedoch eine für heutige Ansprüche unzureichende Polymerlöslichkeit.

Der vorliegenden Erfindung lag daher die Aufgabe zugrunde, hitzestabile, lichtechte und gut polymerlösliche Farbmittel zur Verfügung zu stellen.

Es wurde gefunden, daß die nachstehend definierten Verbindungen der Formel (I) überraschenderweise die gestellte Aufgabe lösen.

Gegenstand der Erfindung ist eine Verbindung der Formel (I) 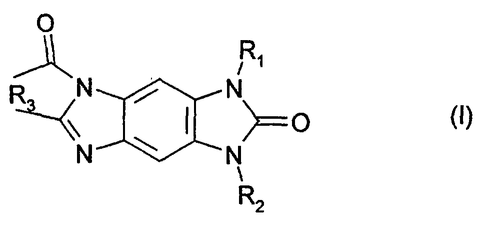 worin
- R¹ und R²: gleich oder verschieden sind und Wasserstoff, C₁-C₆-Alkyl, C₆-C₁₀-Aryl, (C₆-C₁₀)-Aryl-(C₁-C₆)-alkyl oder (C₁-C₆)-Alkyl-(C₆-C₁₀)-aryl bedeuten, wobei die Alkyl- und/oder Arylreste durch Hydroxy, C₁-C₆-Alkoxy, C₆-C₁₀-Aryloxy oder Halogen substituiert sein können,
mit der Maßgabe, daß R¹ und R² nicht gleichzeitig Wasserstoff bedeuten,
- R³: ein Rest der Formel (II) oder (III) ist 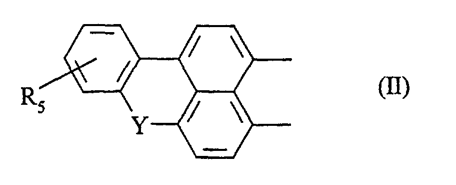
worin
- Y: Schwefel, Sauerstoff oder N-R⁴ bedeutet, worin R4 Wasserstoff, C₁-C₆-Alkyl, C₆-C₁₀-Aryl, (C₆-C₁₀)-Aryl-(C₁-C₆)-alkyl oder (C₁-C₆)-Alkyl-(C₆-C₁₀)-aryl bedeutet, wobei die Alkyl- und/oder Arylreste durch Hydroxy, C₁-C₆-Alkoxy, C₆-C₁₀-Aryloxy oder Halogen substituiert sein können; 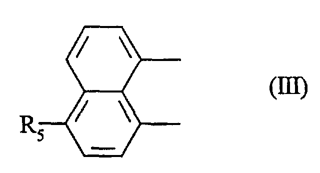
worin
- R⁵: Wasserstoff, Halogen, 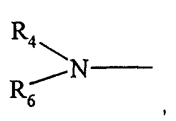 R⁶-0- oder R⁶-Sbedeutet, worin R⁶ C₁-C₆-Alkyl, C₆-C₁₀-Aryl, (C₆-C₁₀)-Aryl-(C₁-C₆)-alkyl oder (C₁-C₆)-Alkyl-(C₆-C₁₀)-aryl ist, worin die Alkyl und/oder Arylreste durch Hydroxy, C₁-C₆-Alkoxy, C₆-C₁₀-Aryloxy oder Halogen substituiert sein können.

Bevorzugt sind Verbindungen der Formel (I), worin R¹ und R² Wasserstoff, Methyl, Ethyl, Propyl, Phenyl, Methylphenyl oder Methoxyphenyl mit der vorstehend genannten Maßgabe bedeuten.

Bevorzugt sind weiterhin Verbindungen der Formel (I), worin R⁴ Wasserstoff, Methyl, Ethyl oder Phenyl bedeutet.

Bevorzugt sind weiterhin Verbindungen der Formel (I), worin R⁵ Wasserstoff, Chlor, Brom, Methoxy, Ethoxy, Phenoxy, -NH-C₆H₄-OCH₃, -O-C₆H₄-OCH₃ oder -S-C₆H₄-OCH₃ bedeutet.

Besonders bevorzugt sind Verbindungen der Formel (I), worin
- R¹: Wasserstoff und
- R²: Methyl, Ethyl, Phenyl oder p-Methoxyphenyl bedeuten.

Besonders bevorzugt sind weiterhin Verbindungen der Formel (I), worin
- R¹: Methyl und
- R²: Methyl, Ethyl, Phenyl oder p-Methoxyphenyl bedeuten.

Gegenstand der vorliegenden Erfindung ist auch ein Verfahren zur Herstellung der Verbindungen der Formel (I), dadurch gekennzeichnet, daß man eine Dicarbonsäure der Formel (IV) 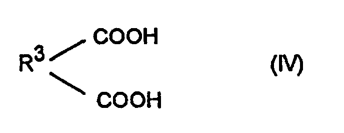 oder, vorzugsweise eines ihrer funktionellen Derivate, z.B. das Säureanhydrid oder -halogenid, mit einem Diamin oder dem Salz eines Diamins der Formel (V) 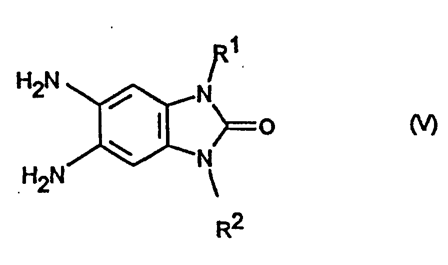 kondensiert.

Als Salze des Diamins der Formel (V) kommen z.B. das Chlorhydrat oder das Sulfat in Betracht.

Die Kondensation erfolgt zweckmäßigerweise in einem molaren Verhältnis (IV):(V) von 0,9:1,1 bis 1,1:0,9.

Die Kondensation kann ohne Lösungsmittel, in der Schmelze, etwa bei Temperaturen zwischen 150°C und 300°C, vorzugsweise bis 250°C, oder in einem inerten Lösungsmittel, bei Temperaturen zwischen 25°C und 300°C, vorzugsweise zwischen 100°C und 250°C, gegebenenfalls in Gegenwart eines Katalysators, durchgeführt werden.

Geeignete Lösungsmittel sind z.B. höhersiedende aliphatische oder aromatische, gegebenenfalls substituierte Kohlenwasserstoffe, z.B. Xylol (Gemisch), Diphenyl, Nitrobenzol, Chlorbenzole, Chlomaphthalin, Glykolether, organische Säuren und Säureamide, insbesondere Dimethylformamid oder N-Methylpyrrolidon. Wird die Dicarbonsäure der Formel (IV) in Form der freien Säure eingesetzt, kann auch Wasser oder ein höher siedender Alkohol, wie Ethylenglykol, als Lösungsmittel verwendet werden.

Als Katalysatoren können z.B. genannt werden: Anorganische oder organische Säuren, z.B. Salzsäure oder Schwefelsäure, Benzol- oder Toluolsulfonsäure, Essigsäure. Auch die Salze organischer Säuren, z.B. Natrium- oder Kaliumacetat eignen sich in manchen Fällen als Katalysatoren.

Im Falle, daß R¹ und R² voneinander verschieden sind, entsteht normalerweise ein Gemisch aller möglichen regioisomeren Verbindungen. Das gleiche gilt sinngemäß wenn unsymmetrische Reste R³ verwendet werden. In den nachfolgenden Beispielen 1 und 2 werden exemplarisch alle möglichen Regioisomere aufgezeigt. Die so hergestellten Gemische brauchen nicht aufgetrennt zu werden und können als Gemisch der erfindungsgemäßen Anwendung zugeführt werden.

Die erfindungsgemäßen Verbindungen eignen sich hervorragend zum Färben von Schmelzen synthetischer polarer Polymere, wie z.B. ABS, Polyester, Polycarbonat oder Polyamide. Polyamide sind z.B. Polykondensate oder Polymerisate aus Dicarbonsäuren und Diaminen, z.B. aus Adipinsäure und Hexamethylendiamin, aus Lactamen, z.B. ε-Caprolactam, oder aus Aminocarbonsäuren, z.B. ω-Aminoundecansäure. Die mit dem Pigment vermischte Polyamidschmelze wird in üblicher Weise verformt, z.B. in Schmelzspinn-, Spritzguß-, Strangspritz- oder Folienblasmaschinen.

Die erfindungsgemäßen Farbstoffe der Formel (I) sind äußerst beständig gegen die bei der Einfärbung der synthetischen Polyamide notwendige Hitzebelastung, die mit ihnen in der Masse gefärbten Substrate zeigen auch ausgezeichnete Echtheitseigenschaften, insbesondere Lichtechtheit. Besonders hervorzuheben ist ihre hohe Polymerlöslichkeit.

Die erfindungsgemäßen Verbindungen der Formel (I) sind auch als Farbmittel in elektrophotographischen Tonern und Entwicklern, wie z.B. Ein- oder Zweikomponentenpulvertonern (auch Ein- oder Zweikomponenten-Entwickler genannt), Magnettoner, Flüssigtoner, Polymerisationstoner sowie Spezialtoner (Lit: L.B. Schein, "Electrophotography and Development Physics"; Springer Series in Electrophysics 14, Springer Verlag, 2nd Edition, 1992) geeignet.

Typische Tonerbindemittel sind Polymerisations-, Polyadditions- und Polykondensationsharze, wie Styrol-, Styrolacrylat-, Styrolbutadien-, Acrylat-, Polyester-, Phenol-Epoxidharze, Polysulfone, Polyurethane, einzeln oder in Kombination, sowie Polyethylen und Polypropylen, die noch weitere Inhaltsstoffe, wie Ladungssteuermittel, Wachse oder Fließhilfsmittel, enthalten können oder im Nachhinein mit diesen Zusätzen modifiziert werden.

Desweiteren sind die erfindungsgemäßen Verbindungen der Formel (I) als Farbmittel in Pulver und Pulverlacken, insbesondere in triboelektrisch oder elektrokinetisch versprühbaren Pulverlacken, die zur Oberflächenbeschichtung von Gegenständen aus beispielsweise Metall, Holz, Kunststoff, Glas, Keramik, Beton, Textilmaterial, Papier oder Kautschuk zur Anwendung kommen (J.F. Hughes, "Electrostatics Powder Coating" Research Studies, JohnWiley & Sons, 1984), geeignet.

Als Pulverlackharze werden typischerweise Epoxidharze, carboxyl- und hydroxylgruppenhaltige Polyesterharze, Polyurethan- und Acrylharze zusammen mit üblichen Härtern eingesetzt. Auch Kombinationen von Harzen finden Verwendung. So werden beispielsweise häufig Epoxidharze in Kombination mit carboxyl- und hydroxylgruppenhaltigen Polyesterharzen eingesetzt. Typische Härterkomponenten (in Abhängigkeit vom Harzsystem) sind beispielsweise Säureanhydride, Imidazole sowie Dicyandiamid und deren Abkömmlinge, verkappte Isocyanate, Bisacylurethane, Phenol- und Melaminharze, Triglycidylisocyanurate, Oxazoline und Dicarbonsäuren.

Außerdem sind die erfindungsgemäßen Verbindungen der Formel (I) als Farbmittel in Ink-Jet- Tinten auf wäßriger und nichtwäßriger Basis sowie in solchen Tinten, die nach dem Hot-melt-Verfahren arbeiten, geeignet.

In den folgenden Beispielen bedeuten Teile Gewichtsteile.

### Beispiel 1

98 Teile 5,6-Diamino-1-methyl-2(3H)-benzimidazolon werden mit 108,9 Teilen Naphthalin-1,8-dicarbonsäureanhydrid in 1200 Teilen Dimethylformamid gemischt. Die Suspension wird 3 Stunden unter Rückfluß erhitzt. Dann werden 160 Teile Dimethylformamid dazu gegeben und Wasser/Dimethylformamid abdestilliert, bis die Temperatur 150°C erreicht hat. Nach einer Stunde wird das Gemisch der Verbindungen der Formeln a und b 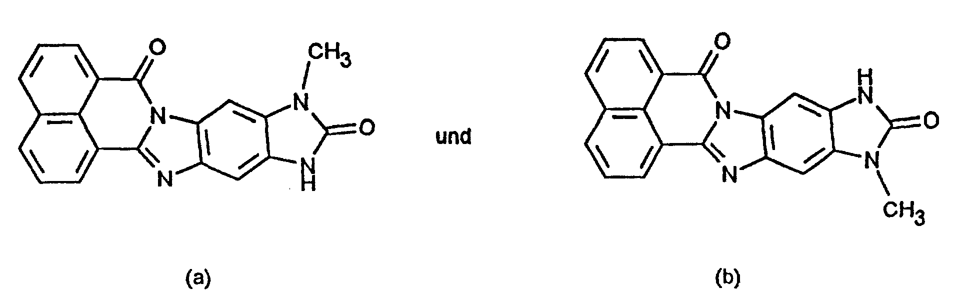 bei 120°C filtriert und mit heißem Dimethylformamid und warmem Wasser gewaschen und getrocknet.
Man erhält 167 Teile eines orangen Pulvers.

### Beispiel 2

133 Teile Benzo-[k,l]-thioxanthene-3,4-dicarbonsäureanhydrid werden in 1600 Teilen N-Methylpyrrolidon und 150 Teilen Essigsäure suspendiert. 78 Teile 5,6-Diamino-1-methyl-2(3H)-benzimidazolon werden bei Raumtemperatur beigefügt. Die Suspension wird 3 Stunden unter Rückfluß erhitzt. Nach 2 Stunden wird das Gemisch der Produkte der Formeln c, d, e und f 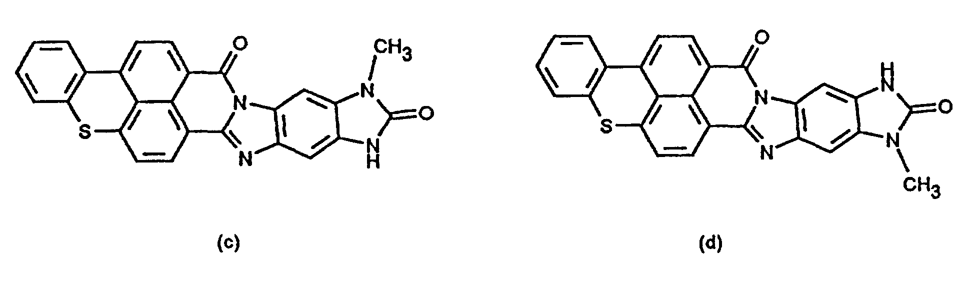 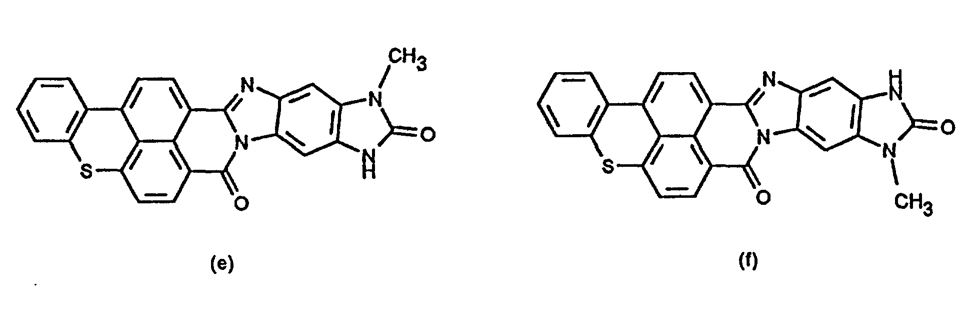 bei 120°C filtriert und mit heißem N-Methylpyrrolidon und mit Methanol gewaschen und getrocknet.

Man erhält 164 Teile eines dunkelroten Pulvers.

Die in der nachstehenden Tabelle 1 aufgeführten Farbstoffe werden analog zu Beispiel 2 hergestellt.

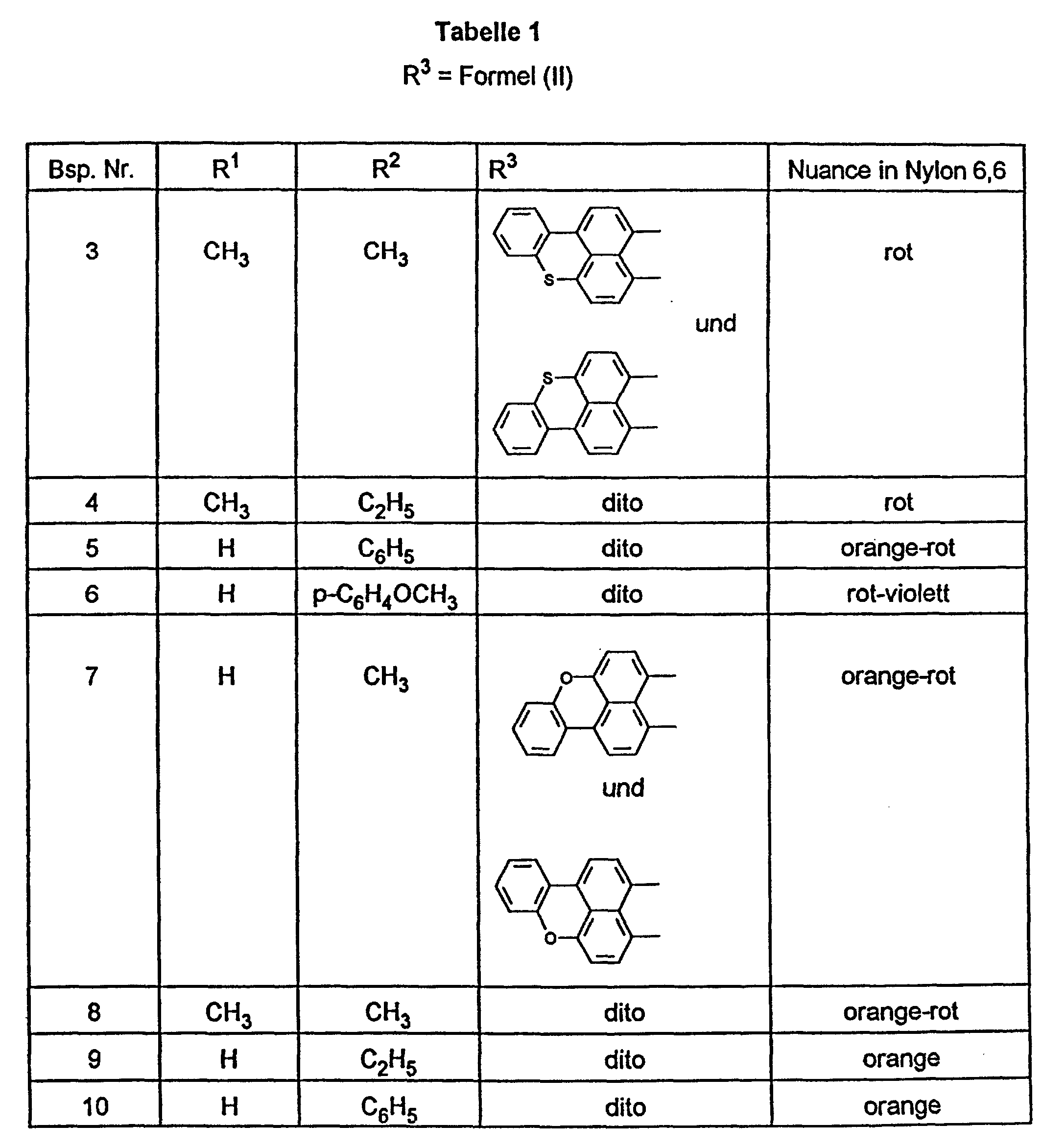

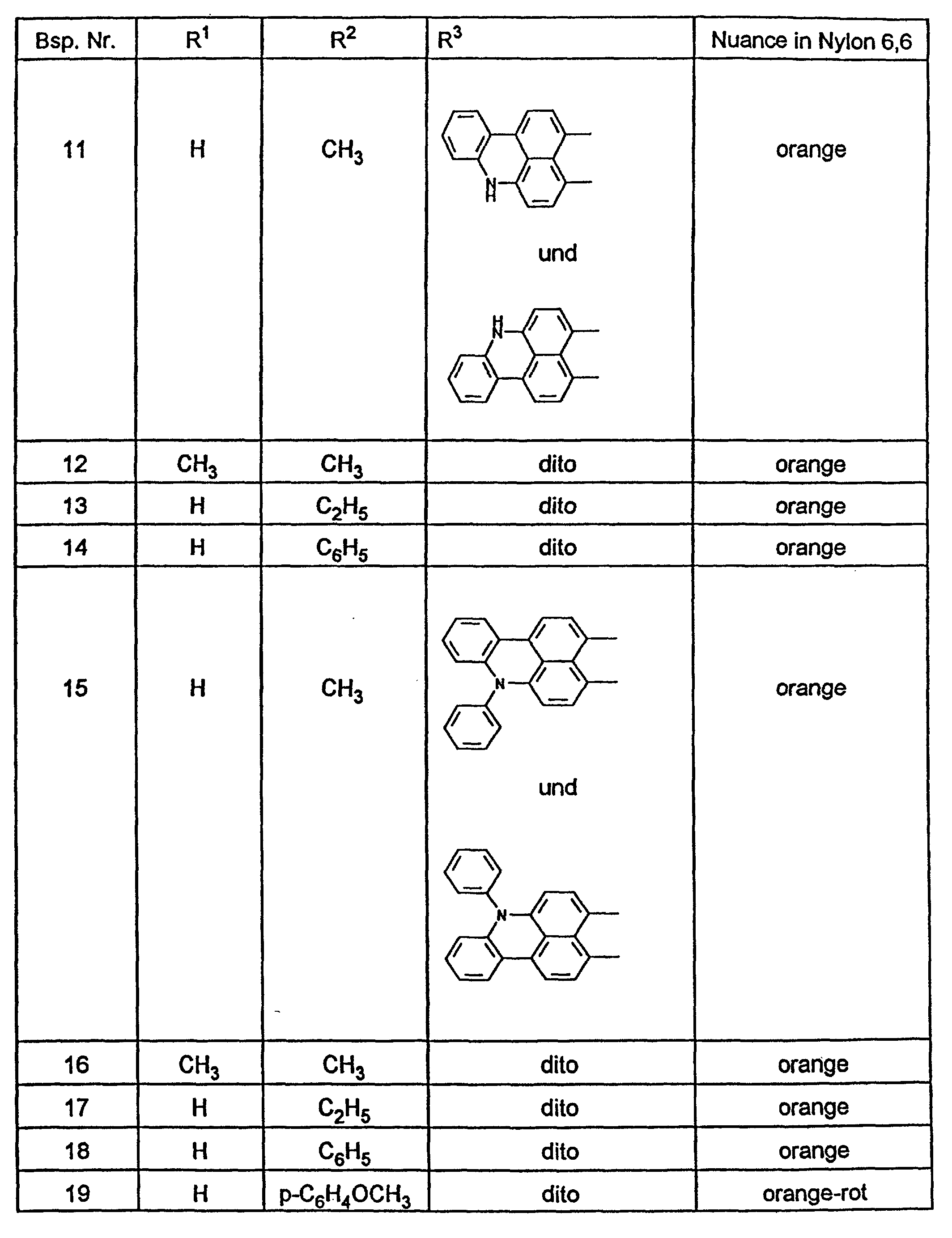

Die in der nachstehenden Tabelle 2 aufgeführten Farbstoffe werden analog zu Beispiel 1 hergestellt.

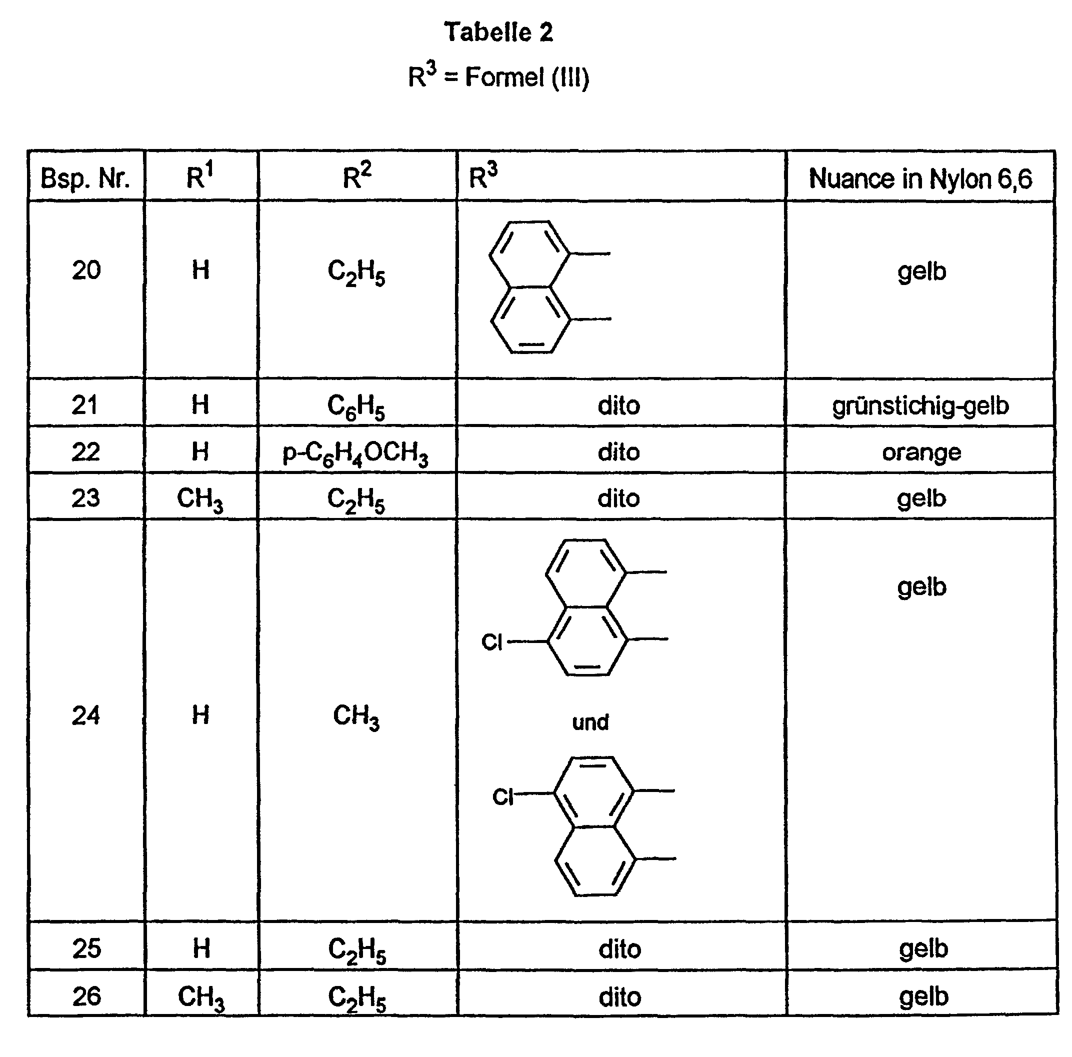

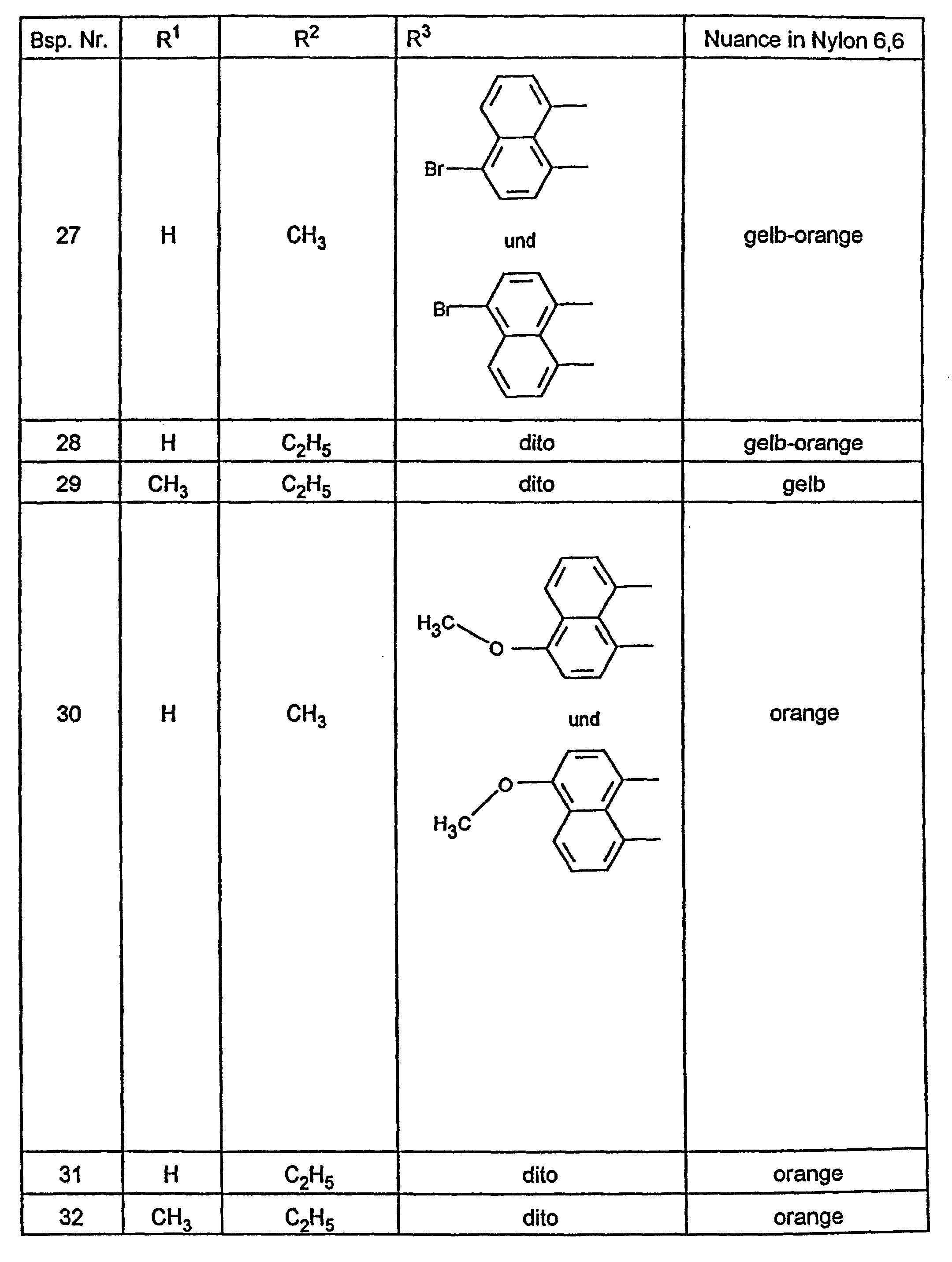

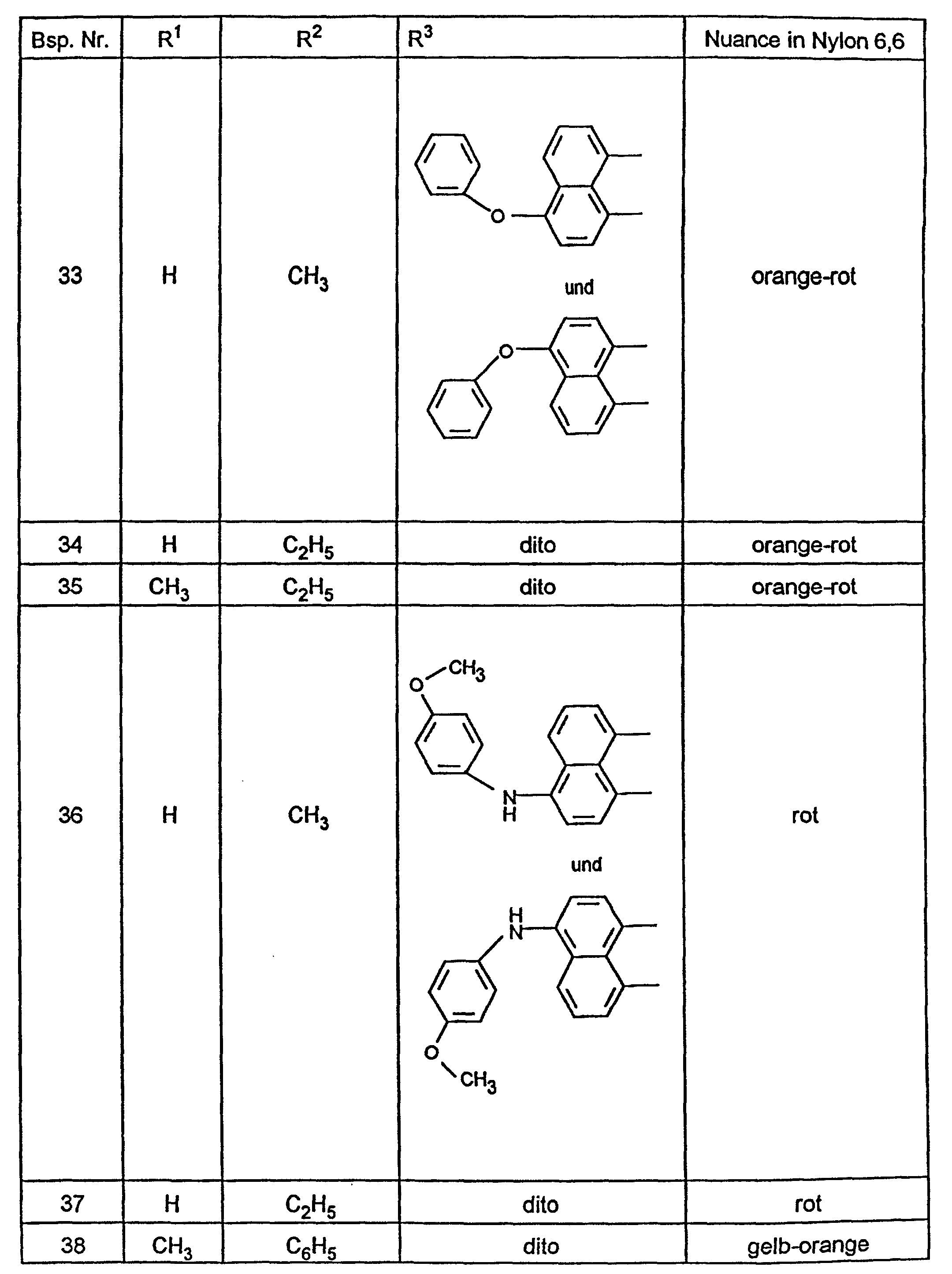

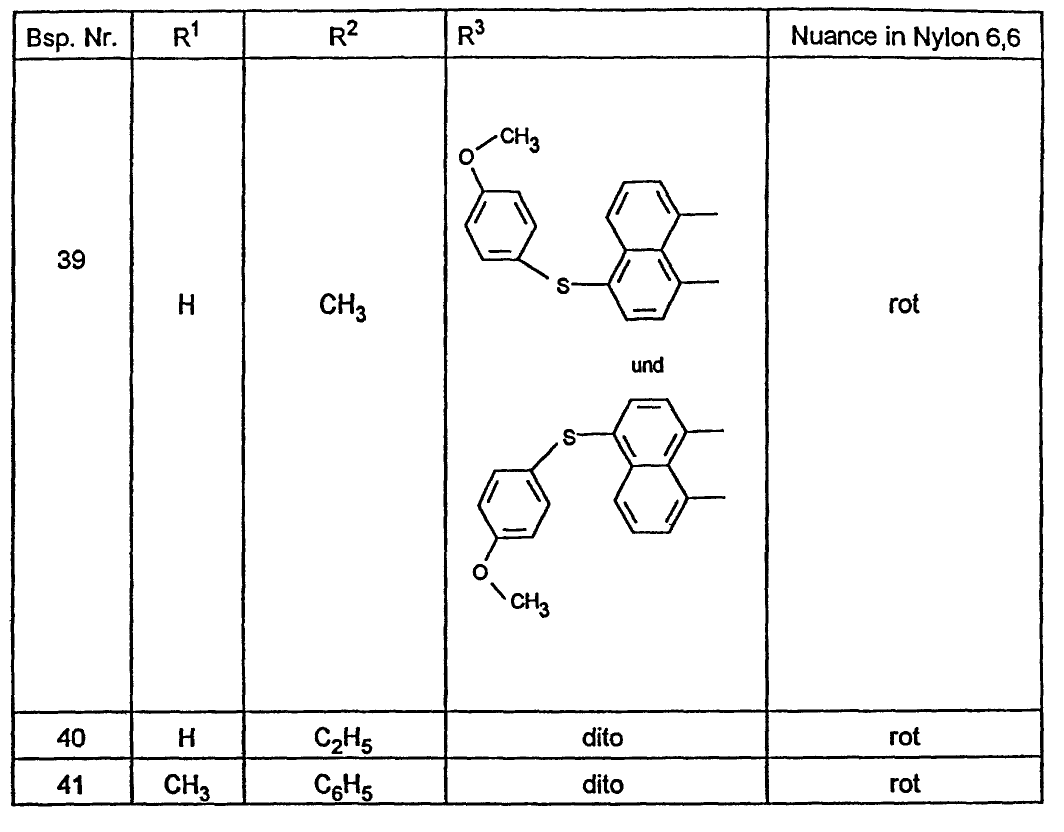

### Anwendungsbeispiel

100 Teile Polycaprolactam in Form eines Pulvers werden mit 0,1 , resp. 1,0 Teilen des Farbstoffes aus Beispiel 1 in Pulverform in einem Trommelmischer vermischt. Das Pulver verteilt sich nach kurzer Zeit gleichmäßig und haftet auf dem Granulat. Nach ca. 10 Minuten wird das Gemisch 16 Stunden bei 120°C getrocknet, in eine Schmelzspinnmaschine gebracht und nach ca. 8-minütiger Verweilzeit bei 275 - 280°C unter Stickstoffatmosphäre zu Fasern versponnen. Die gelb gefärbten Fasern sind außerordentlich lichtecht.

Auf dieselbe Weise können auch alle übrigen bekannten synthetischen Polyamide (Nylon, Perlon etc.) sowie Polyester, ABS, Polycarbonate mit sämtlichen Verbindungen der Beispiele 1-41 in der Masse gefärbt werden.

## Patentansprüche

1. Verbindung der Formel (I) 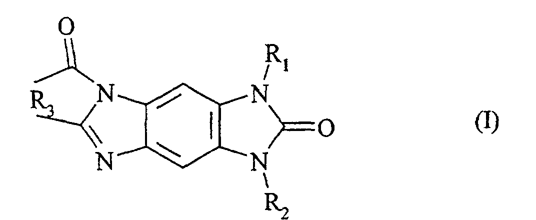 worin
R¹ und R² gleich oder verschieden sind und Wasserstoff, C₁-C₆-Alkyl, C₆-C₁₀-Aryl, (C₆-C₁₀)-Aryl-(C₁-C₆)-alkyl oder (C₁-C₆)-Alkyl-(C₆-C₁₀)-aryl bedeuten, wobei die Alkyl- und/oder Arylreste durch Hydroxy, C₁-C₆-Alkoxy, C₆-C₁₀-Aryloxy oder Halogen substituiert sein können,
mit der Maßgabe, daß R¹ und R² nicht gleichzeitig Wasserstoff bedeuten,
R³ ein Rest der Formel (II) oder (III) ist 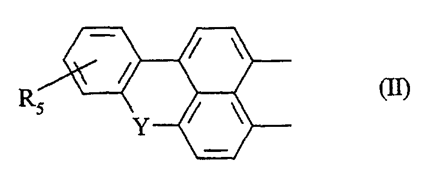 worin Y Schwefel, Sauerstoff oder N-R⁴ bedeutet, worin R⁴ Wasserstoff, C₁-C₆-Alkyl, C₆-C₁₀-Aryl, (C₆-C₁₀)-Aryl-(C₁-C₆)-alkyl oder (C₁-C₆)-Alkyl-(C₆-C₁₀)-aryl bedeutet, wobei die Alkyl- und/oder Arylreste durch Hydroxy, C₁-C₆-Alkoxy, C₆-C₁₀-Aryloxy oder Halogen substituiert sein können; 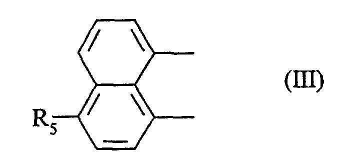 worin
R⁵ Wasserstoff, Halogen, 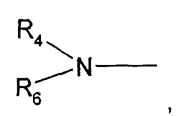 R⁶-O- oder R⁶-S- bedeutet, worin R⁶ C₁-C₆-Alkyl, C₆-C₁₀-Aryl, (C₆-C₁₀)-Aryl-(C₁-C₆)-alkyl oder (C₁-C₆)-Alkyl-(C₆-C₁₀)-aryl ist, worin die Alkyl- und/oder Arylreste durch Hydroxy, C₁-C₆-Alkoxy, C₆-C₁₀-Aryloxy oder Halogen substituiert sein können.

2. Verbindung nach Anspruch 1, **dadurch gekennzeichnet, daß** R¹ und R² Wasserstoff, Methyl, Ethyl, Propyl, Phenyl, Methylphenyl oder Methoxyphenyl bedeuten.

3. Verbindung nach Anspruch 1 oder 2, **dadurch gekennzeichnet, daß** R⁴ Wasserstoff, Methyl, Ethyl oder Phenyl bedeutet.

4. Verbindung nach einem der Ansprüche 1 bis 3, **dadurch gekennzeichnet, daß** R⁵ Wasserstoff, Chlor, Brom, Methoxy, Ethoxy, Phenoxy, -NH-C₆H₄-OCH₃, -O-C₆H₄-OCH₃ oder -S-C₆H₄-OCH₃ bedeutet.

5. Verbindung nach einem der Ansprüche 1 bis 4, **dadurch gekennzeichnet, daß**
R¹ Wasserstoff und
R² Methyl, Ethyl, Phenyl oder p-Methoxyphenyl bedeuten.

6. Verbindung nach einem der Ansprüche 1 bis 4, **dadurch gekennzeichnet, daß**
R¹ Methyl und
R² Methyl, Ethyl, Phenyl oder p-Methoxyphenyl bedeuten.

7. Verfahren zur Herstellung einer Verbindung nach einem der Ansprüche 1 bis 6, **dadurch gekennzeichnet, daß** man eine Dicarbonsäure der Formel (IV) 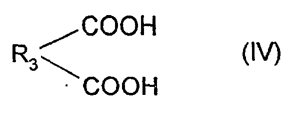 oder eines ihrer funktionellen Derivate mit einem Diamin oder dem Salz eines Diamins der Formel (V) 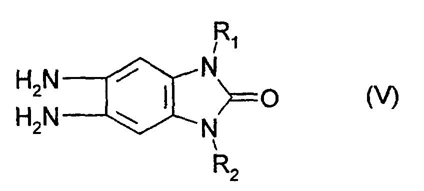 kondensiert.

8. Verfahren nach Anspruch 7, **dadurch gekennzeichnet, daß** das funktionelle Derivat der Dicarbonsäure das Säureanhydrid oder Säurechlorid ist.

9. Verwendung einer Verbindung nach einem der Ansprüche 1 bis 6 zum Färben von synthetischen Polyamiden, Polyestern, ABS oder Polycarbonaten sowie als Farbmittel in elektrophotographischen Tonern und Entwicklern, in Pulverlacken und in Ink-Jet-Tinten.

10. Verwendung nach Anspruch 9 zum Massefärben von Polykondensaten aus Dicarbonsäuren und Diaminen, aus Lactamen oder aus Aminocarbonsäuren.

## Claims

1. Compound of the formula (I) 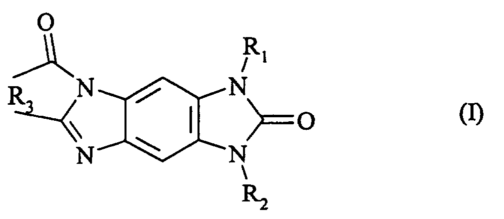 where
R¹ and R² are the same or different and are each hydrogen, C₁-C₆-alkyl, C₆-C₁₀-aryl, (C₆-C₁₀)-aryl-(C₁-C₆)-alkyl or (C₁-C₆)-alkyl-(C₆-C₁₀)-aryl, wherein the alkyl and/or aryl radicals may be substituted by hydroxyl, C₁-C₆-alkoxy, C₆-C₁₀-aryloxy or halogen,
with the proviso that R¹ and R² are not both hydrogen,
R³ is a radical of the formula (II) or (III) 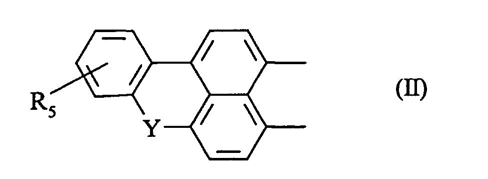 where
Y is sulphur, oxygen or N-R⁴, where R⁴ is hydrogen, C₁-C₆-alkyl, C₆-C₁₀-aryl, (C₆-C₁₀)-aryl-(C₁-C₆)-alkyl or (C₁-C₆)-alkyl-(C₆-C₁₀)-aryl, wherein the alkyl and/or aryl radicals may be substituted by hydroxyl, C₁-C₆-alkoxy, C₆-C₁₀-aryloxy or halogen; 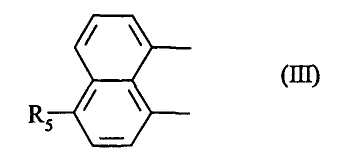
where
R⁵ is hydrogen, halogen, 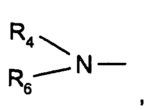 R⁶-O- or R⁶-S-, where R⁶ is C₁-C₆-alkyl, C₆-C₁₀-aryl, (C₆-C₁₀)-aryl-(C₁-C₆)-alkyl or (C₁-C₆)-alkyl-(C₆-C₁₀)-aryl, wherein the alkyl and/or aryl radicals may be substituted by hydroxyl, C₁-C₆-alkoxy, C₆-C₁₀-aryloxy or halogen.

2. Compound according to Claim 1, **characterized in that** R¹ and R² are each hydrogen, methyl, ethyl, propyl, phenyl, methylphenyl or methoxyphenyl.

3. Compound according to Claim 1 or 2, **characterized in that** R⁴ is hydrogen, methyl, ethyl or phenyl.

4. Compound according to any one of Claims 1 to 3, **characterized in that** R⁵ is hydrogen, chlorine, bromine, methoxy, ethoxy, phenoxy, -NH-C₆H₄-OCH₃, -O-C₆H₄-OCH₃ or -S-C₆H₄-OCH₃.

5. Compound according to any one of Claims 1 to 4, **characterized in that** R¹ is hydrogen and R² is methyl, ethyl, phenyl or p-methoxyphenyl.

6. Compound according to any one of Claims 1 to 4, **characterized in that** R¹ is methyl and R² is methyl, ethyl, phenyl or p-methoxyphenyl.

7. A process for preparing a compound according to any one of Claims 1 to 6, **characterized in that** a dicarboxylic acid of the formula (IV) 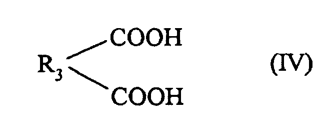 or one of its functional derivatives is condensed with a diamine or the salt of a diamine of the formula (V)

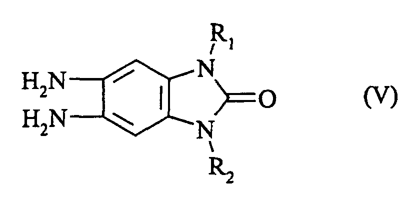

8. Process according to Claim 1, **characterized in that** the functional derivative of the dicarboxylic acid is the anhydride or acyl chloride.

9. Use of a compound according to any one of Claims 1 to 6 for colouring synthetic polyamides, polyesters, ABS or polycarbonates and also as a colorant in electrophotographic toners and developers, in powder coatings and in ink jet inks.

10. Use according to Claim 9 for mass coloration of polycondensates formed from dicarboxylic acids and amines, from lactams or from amino carboxylic acids.

## Revendications

1. Composé de (formule) (I) 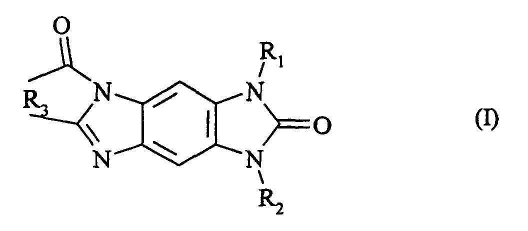 dans laquelle
R¹ et R² sont identiques ou différents et représentent l'hydrogène, un alkyle en C₁ à C₆, un aryle en C₆ à C₁₀, un alkyle en C₁ à C₆ - aryle en C₆ à C₁₀ ou un aryle en C₆ à C₁₀ - alkyle en C₁ à C₆, les radicaux alkyle et/ou aryle pouvant être substitués avec un groupe hydroxy, alkoxy en C₁ à C₆, aryloxy en C₆ à C₁₀ ou un halogène,
avec la condition que R¹ et R² ne représentent pas tous deux l'hydrogène,
R³ représentant un radical de formule (II) ou (III) 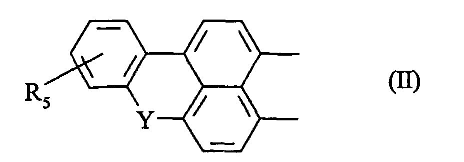 dans laquelle
Y représente le soufre, l'oxygène ou N-R⁴, R⁴ représentant l'hydrogène, un alkyle en C₁ à C₆, un aryle en C₆ à C₁₀, un alkyle en C₁ à C₆ - aryle en C₆ à C₁₀ ou un aryle en C₆ à C₁₀-alkyle en C₁ à C₆, les radicaux alkyle et/ou aryle pouvant être substitués avec un groupe hydroxy, alkoxy en C₁ à C₆, aryloxy en C₆ à C₁₀ ou un halogène, 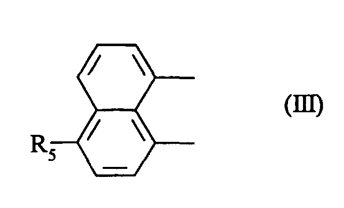
dans laquelle
R⁵ représente l'hydrogène, un halogène, 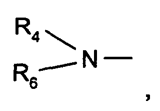 R⁶-O- ou R⁶-S-, R⁶ représentant un alkyle en C₁ à C₆, un aryle en C₆ à C₁₀, un alkyle en C₁ à C₆ - aryle en C₆ à C₁₀ ou un aryle en C₆ à C₁₀ - alkyle en C₁ à C₆, les radicaux alkyle et/ou aryle pouvant être substitués avec un groupe hydroxy, alkoxy en C₁ à C₆, aryloxy en C₆ à C₁₀ ou un halogène.

2. Composé selon la revendication 1, **caractérisé en ce que** R¹ et R² représentent l'hydrogène, le méthyle, l'éthyle, le propyle, le phényle, le méthylphényle ou le méthoxyphényle.

3. Composé selon la revendication 1 ou 2, **caractérisé en ce que** R⁴ représente l'hydrogène, le méthyle, l'éthyle ou le phényle.

4. Composé selon l'une quelconque des revendications 1 à 3, **caractérisé en ce que** R⁵ représente l'hydrogène, le chlore, le brome, un groupe méthoxy, éthoxy, phénoxy, -NH-C₆H₄-OCH₃, -O-C₆H₄-OCH₃ ou -S-C₆H₄-OCH₃.

5. Composé selon l'une quelconque des revendications 1 à 4, **caractérisé en ce que** R¹ représente l'hydrogène et R² représente le méthyle, l'éthyle, le phényle ou le p-méthoxyphényle.

6. Composé selon l'une quelconque des revendications 1 à 4, **caractérisé en ce que** R¹ représente le méthyle et R² représente le méthyle, l'éthyle, le phényle ou le p-méthoxyphényle.

7. Procédé pour la préparation d'un composé selon l'une quelconque des revendications 1 à 6, **caractérisé en ce que** l'on condense un acide dicarboxylique de formule (IV) 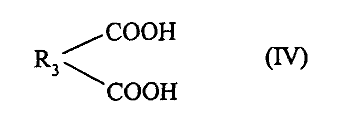 ou l'un de ses dérivés fonctionnels avec une diamine ou le sel d'une diamine de formule (V)

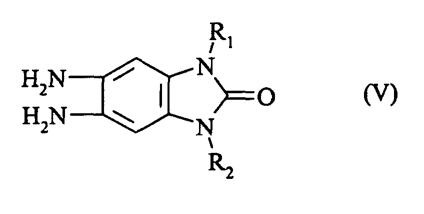

8. Procédé selon la revendication 7, **caractérisé en ce que** le dérivé fonctionnel de l'acide dicarboxylique est l'anhydride acide ou le chlorure d'acide.

9. Utilisation d'un composé selon l'une quelconque des revendications 1 à 6 pour la coloration de polyamides, de polyesters, d'ABS ou de polycarbonates de synthèse, ainsi que comme colorant dans des toners et développeurs électrophotographiques, dans des peintures en poudre et dans des encres pour impression à jet d'encre.

10. Utilisation selon la revendication 9, pour la coloration de la masse de polycondensats d'acides dicarboxyliques et de diamines, de lactames ou d'acides aminocarboxyliques.
